# EUROPEAN PATENT APPLICATION

(11) **EP 0 923 935 A1**
(43) Date of publication of application: **23.06.1999**
(21) Application number: 97903583.9
(22) Date of filing: 20.02.1997
(51) Int. Cl.: A61K 31/70, A61K 33/06, A61K 9/06, A61K 9/08, A61K 9/12

(54) **EXTERNAL PREPARATION FOR CURING LESIONED TISSUES**

(30) Priority: 23.02.1996 JP 3593096
(71) Applicant: Chuyakuken, Ltd., Naha-shi, Okinawa 900 (JP)
(72) Inventor: OKU, Kinuko, Chuyakuken, Ltd., Naha-shi, Okinawa 900 (JP)
(74) Representative: Türk, Gille, Hrabal
(86) International application number: JP9700454
(87) International publication number: WO9730711

(57) **Abstract**

A locally administrable preparation for treating affected tissues effective particularly for the affected tissues caused in the rectum or the anus, which contains a pharmaceutically effective component compounds including tannic acid and potassium aluminum sulfate and a base component for use with the locally administrable preparation. The locally administrable preparation is in the form of, for example, ointment, cream, and suppository.

## Description

### TECHNICAL FIELD

The present invention relates to a locally administrable preparation for treating affected tissues and, more particularly, to a locally administrable preparation effective for treating or curing affected tissues, especially the affected tissues of the rectum or the anus.

### BACKGROUND TECHNOLOGY

As the affected tissues of the rectum or the anus as referred to herein, there may be mentioned, for example, those affected tissues caused by the hemorrhoid resulting from the grumous extension of the hemorrhoidal venous plexus located at the lower portion of the rectum or under the mucous membranes of the anus. Many patients with the hemorrhoid are always suffering from hemafecia, hemorrhoidal pain, prolapse of the internal hemorrhoid, inflammation of the outer hemorrhoid, and mucous hemafecia and from other symptoms. These symptoms may interfere with the work for many hemorrhoidal patients.

As the diseases of the rectum and the anus, there may be enumerated more than ten. First, they may be broken down into germfree and germ-originated by the cause of the diseases. Further, they may be broken down into specific and non-specific by the mechanism of the diseases. Further, based on the site at which the pathological changes occur, they may be divided into a mucous zone (an indolent zone) and an anal canal cortex zone (a pain zone). In addition, they may be broken down on the basis of the pathological lesions into congestion, exudation, edema and so on, caused by germ-originated inflammation or germ-free inflammation. Based on the functionality and the organopathy of the anal canal, they may also be divided into the clogging of the faece and the constipation to be caused by the narrowing of the rectal canal or the anal canal and into the prolapse of the internal hemorrhoid, prolapse of the mucous membrane or mucus flux to be induced by the relaxation of the anal canal. Accordingly, upon treatment of the affected tissues of the rectum and the anus, the kinds of the diseases as described hereinabove also have to be taken into consideration.

As the method for treating the affected tissues at the lower portion of the rectum and the anus, a variety of processes can be adopted. The treatment processes such as immersing in a warm bath, coating with an ointment, inserting a suppository and so on can be applied, on the one hand, to patients with hemorrhoidal symptoms in a light condition. The treatment processes such as, for example, by injection into the hemorrhoidal node or by a surgical operation of removing the hemorrhoid may be adopted, on the other hand, to patients with hemorrhoidal symptoms in a severe condition. All the treatment processes but the surgical operation of removing the hemorrhoid are each merely a symptomatic therapy. Further, the treatment processes by the surgical operation cause intolerable pain particularly after the operation so that, as a course of matter, the patients suffering from the hemorrhoid wish to avoid the treatment by such a surgical operation if they could. Moreover, if the hemorrhoid could be treated without causing any pain, it is an extremely good news to the patients suffering from the hemorrhoid.

As medicines to be applied to the treatment of the affected tissues at the lower portion of the rectum or the anus, such as the hemorrhoid, by a non-surgical method other than the treatment thereof by removal of the hemorrhoidal portion by the surgical operation, there may be applied a locally adminstrable preparation such as, for example, a topical preparation such as an ointment or the like, or a local preparation such as, for example, a suppository or the like. The local or topical preparations such as ointments, on the one hand, may be applied to the local hemorrhoid and so on, i.e. to the lesions exposed to the outside of the fundament. The local preparations such as suppositories and so on, on the other hand, may be applied to the internal hemorrhoid and so on, i.e. to the lesions located at the lower portion of the rectum or in the inside of the anus. However, a number of ointments and suppositories commercially available as the locally adminstrable preparations for treating the affected tissues, such as hemorrhoid, is so small that only three or four kinds of local or topical preparations are applied in a usual case. Among them, major local or topical preparations are formulated each by a traditional prescription for Chinese medicines. Further, an ointment contains chrysanthemum pollen (the powder formed in a composite plant) as a basic component, with talc, moonstone (borax), animal bone powders (animal bone fossil powders), white spruce, borneol, and vermilion. The such ointment can provide mainly the pharmacological action of stopping bleeding and contracting tissues of the body without effects upon control over bacteria, elimination of inflammation and alleviation of pain. The greatest issue inherent in the such ointment, however, is that it contains mercury. There also has been conventionally employed a suppository having the same composition as this ointment but without the use of the mercuric component, which is further prepared by increasing or decreasing the amounts of the rest of the composition and adding musk thereto. This suppository is a traditional Chinese medicine and has been extensively employed as a local or topical preparation for the rectum and the anus. This suppository, however, has the demerits that the musk employed as a component cannot be employed any more as a component for medicine due to the national regulations. What is further at issue for the suppository is that it lacks not only experimental data but also clinical data, which are worthwhile to rely on.

As the method for curing the affected part from the source of the diseases other than the method of removing the affected tissues caused by the hemorrhoid or the like at the lower portion of the rectum or the anus by a surgical operation, it is proposed that the affected lesions are administered by injection of a medical preparation, the affected tissues of the affected lesions are caused to sclerose and the scleroses part of the affected tissues injected is allowed to be removed naturally from the normal tissues. As such a medicinal preparation to be employed for such pharmacotherapy, there is proposed an agent called "XIAOZHILING" in Chinese. The composition of this agent comprises an aqueous solution containing tannic acid, alum (potassium aluminum sulfate), sodium citrate, dextran, glycerin and trichlorobutyl alcohol. This solution, however, cannot be said to be preferred for injection because it contains trichlorobutyl alcohol.

Thus, there has been proposed a sclerosing agent for treating affected tissues having a preferred composition by improving this injection. Japanese Patent Laid-open Application No. 4-225,920 of the application filed in the name of the present applicant discloses a sclerosing agent containing tannic acid, alum, sodium citrate, sodium hydrogen sulfite, dextran, glycerin and a stabilizer prepared from plant extract. This sclerosing agent, however, suffers from the disadvantage that it is difficult to formulate injectable preparations having always the required constant quality because they use such a plant extract as a stabilizer.

Therefore, the present applicants have made extensive research and studies with the attempt to provide a sclerosing agent having the basic composition as described hereinabove yet having a constant quality and a sufficient degree of stability as a preparation. As a result, it has been found that a remarkably high level of stability as a preparation and a persistently constant quality can be achieved by a sclerosing agent prepared as an injectable agent without addition of the stabilizer obtained from the plant extract, that is, by ingeniously designing the order of formulating tannic acid and alum (potassium aluminum sulfate). This injectable preparation basically comprises tannic acid, potassium aluminum sulfate, sodium citrate and sodium hydrogen sulfite. The details of this injectable preparations are described in International Publication No. WO94/06443 and the counterpart patent publications.

As this sclerosing agent for treating the affected tissues is an injectable preparation, it is obvious that the administration of the injectable preparation gives some degree of pain upon treatment. Further, the treatment by medical doctors is also inevitable upon treatment. These matters are painful and inconvenient to patients suffering from the hemorrhoid or other related diseases. Demands have therefore been made for a long time, accordingly, that an agent for treating the affected tissues resulting from the hemorrhoid, which can be employed in a simplified manner even at home for treatment by patients themselves with the symptom in a light condition.

On the other hand, for patients in a grave condition of the disease, the treatment after the removal of the affected tissues by a surgical operation is also extremely painful. Further, the lesions affected by the hemorrhoid are very likely to be infected with bacteria and it is very difficult to keep always clean. In addition, the affected lesions are located in the anal canal, a severe level of pain is caused to occur at the time of defecation or in cases where the surgically treated portion becomes dirty, and such pain continues for a while.

As an alternative method to meet with such a demand, there may be applied, for example, the method for administering the sclerosing agent for treating the affected tissues as a locally administrable agent to the anal and/or rectal lesions. As the such locally administrable agent there may be employed, for example, a topical preparation such as an ointment, etc. or a local preparation such as a suppository, etc. The local or topical preparations may be applied in various preparation forms. The locally or topically administrable preparations may be in the form of, for example, ointments, creams, lotions, and the like, and the suppositories may be in the form, for example, in each of which the pharmaceutically active components are dissolved and dispersed in a solid oily material such as cacao butter, witepsol or the like, in which the pharmaceutically active components are dissolved and dispersed in a hydrophilic base material such as macrogols or the like, or in the form of a gelatin capsule, for example, in which the pharmaceutically active components are dissolved and dispersed in a liquid oil such as a triglyceride of a medium- or large-chain fatty acid, a vegetable oil or the like, and the resulting dispersion is filled in capsules covered with a gelatin film.

The rectal administration of the pharmaceutically active compositions as locally administrable preparations such as suppositories provides various advantages and features even when they are administered in either of the various preparation forms. Further, even in the event where the administration by injection may otherwise induce the risk of causing some side effects, such rectal administration can in many cases prevent the risk of an occurrence of such side effects. In other words, even in cases where some pharmaceutically active compositions would induce the risk of causing some side effects such as, for example, contructure of the muscle when they are administered through a percutaneous route such as, for example, by an injection administration, the risk of causing any such side effects can be avoided in many occasions if they would be administered through a rectal route such as, for example, in the form of a suppository.

Further, the forms of administration, such as rectal administration, can provide the advantages that the administration can be made in a relatively easy way without failure, compared with the other forms of administration such as administration through a percutaneous route and that less limitations are imposed on the time of administration. Furthermore, the rectal administration with suppositories or the like can be made relatively easily and in a sure way even after operation. In addition, the administration through the rectum or the like can provide one of the greatest merits that no pain can be caused upon administration, unlike by injection.

The rectal administration of the local preparations in the form of suppositories or the like can further present the advantages and features that in many cases the total rate of absorption of the pharmaceutically active components of the preparations can be increased to a higher level. In other words, unlike the stomach and so on, the rectum has little or no acids or enzymes that may otherwise decompose the pharmaceutically active components thereof. Further, such rectal administration does not impose limitations upon the timing of administration such as, for example, before or after meal or between meals. As the local or topical administration, for example, by the rectal administration does not give any pain, it can be carried out easily even to infants and children to whom it is otherwise found difficult in many cases to perform the administration by injection. In addition, the local administration through a rectal route or through such other routes can provide the advantages and features that the pharmaceutically active components can be absorbed at a milder speed and that the effects of the pharmaceutically active components can be sustained for a longer period, as compared particularly with the administration by injection and so on.

### DISCLOSURE OF THE INVENTION

Therefore, the present invention has the object to provide a locally administrable preparation in a preparation form comprising, for example, a form of a topical or local preparation such as an ointment or a suppository form, as a sclerosing agent for treating the affected tissues of the rectum and/or the anus resulting from the diseases such as hemorrhoid or the like. The locally administrable preparations according to the present invention are not only effective for treating the affected tissues originated from the hemorrhoid in a light condition, but also for preventing pain to be induced or caused mainly by the constriction of the constrictor muscle after operation. Further, they can control the growth of bacteria and eliminate inflammation, reduce a swelling, stop pain, accelerate the normal and rapid growth of the blastocyte, and facilitate the suture of a wound, each of such symptoms being caused in association with the combination that may be caused after the surgery of the rectum or the anus.

The locally administrable preparation provided by the present invention as a sclerosing agent for treating the affected tissues is a locally administrable preparation for treating the affected tissues of the anus and/or the rectum, which is so useful for patients themselves to administer it even at home in accordance with prescribed procedures and prescriptions by the medical doctor.

The locally administrable preparation for treating the affected tissues according to the present invention contains a pharmaceutically active component and a base component for a locally administrable preparation, and the pharmaceutically active component comprises tannic acid and potassium aluminum sulfate.

In the specification, the term "pharmaceutically active component compound(s)" and related terms referred to herein are intended to mean tannic acid and(or) potassium aluminum sulfate acting as major active components for the locally administrable preparation for treating the affected tissues according to the present invention, unless otherwise stated herein or as is apparent from the context of this specification.

For the locally administrable preparations for treating the affected tissues according to the present invention, tannic acid has in itself a somewhat strong action of contracting the tissues and it can work as alleviating or reducing the congestion of the mucous membrane and the bleeding. Further, tannic acid has the action of controlling and killing bacteria including both gram-negative and gram-positive bacteria. On the other hand, potassium aluminum sulfate can release aluminum ions in a solution state or in an aqueous state and has a relatively strong action of stopping the bleeding and contracting the body tissues. Further, it can act as accelerating the normal growth of the blastocyte at the site of a wound. Therefore, by formulating tannic acid and potassium aluminum sulfate at an appropriate rate, the preparations according to the present invention can provide the anti-inflammatory action of competing with inflammation resulting from the hemorrhoid or other diseases and the anti-exudation action against exudation resulting therefrom, the action of alleviating inflammation, as well as the action of stopping the bleeding and reducing a swelling.

For the local or topical preparations for treating the affected tissues according to the present invention, the preparation forms of the topical preparations may include, for example, ointments, creams, lotions, aerosols, and the like, and the preparation forms of the other preparations may include, for example, suppositories and the like. Moreover, it is preferred that the base components for the local or topical preparations may be selected appropriately in accordance with the forms of the preparations. It is therefore preferred that the compositions of the local or topical preparations according to the present invention are prepared appropriately in accordance with the forms of the local or topical preparations.

In the preparations for sclerosing the affected tissues according to the present invention, in each of the preparation forms, the pharmaceutically active component compounds in the local or topical preparations are contained in a dissolved state in a liquid or solid solvent as a base component demonstrating compatibility with the pharmaceutically active component compounds. Potassium aluminum sulfate acting as one of the pharmaceutically active component compounds is readily soluble in water and little soluble in ethanol or the like. On the other hand, tannic acid acting as the other of the pharmaceutically active component compounds is readily soluble in water, ethanol, acetone or the like. It is to be noted herein that sufficient care should be paid to the order or manner of formulating potassium aluminum sulfate with tannic acid, when potassium aluminum sulfate is formulated with tannic acid, particularly when potassium aluminum sulfate is formulated with tannic acid each in a liquid state. In particular, the reason why careful attention has to be paid to the order or manner of formulating the components is because, when potassium aluminum sulfate is dissolved in water, aluminum ions are caused to be released in an aqueous state from the potassium aluminum sulfate upon decomposition and they are caused to precipitate in the aqueous solution upon reaction with the tannic acid and that this phenomenon causes a loss of the effects of tannic acid. Further, in order to dissolve the pharmaceutically active component compounds in an organic solid compound, the organic solid compound is thermally molten and the pharmaceutically active component compounds are added to the molten material and mixed therewith or the pharmaceutically active component compounds are mixed with the organic solid compound and the resulting mixture is thermally molten. The pharmaceutically active component compounds in the local or topical preparations according to the present invention are preferably present in a liquid or solution state at ambient temperature.

It is preferred that the amount of the pharmaceutically active component compounds to be employed in the sclerosing agents for treating the affected tissues according to the present invention may be changed in a various way in accordance with the kind of the pharmaceutically active component compounds, the forms of the local or topical preparations, sex and ages of patients, symptoms of the diseases, and so on. When the application form of the preparation is in the form of the topical preparations, such as an ointment or the like, potassium aluminum sulfate of the pharmaceutically active component compounds may be formulated with tannic acid at a rate of approximately 0.1% to 30% by weight, preferably from approximately 1% to 20% by weight, more preferably from approximately 5% to 10% by weight, with respect to the weight of the topical preparation. On the other hand, the rate of the tannic acid as the other one of the pharmaceutically active component compounds may range from approximately 10% to 90% by weight, preferably from approximately 30% to 70% by weight, more preferably from approximately 40% to 60% by weight, on the basis of the weight of potassium aluminum sulfate.

The base components to be employed in preparing the locally administrable preparations for treating the affected tissues according to the present invention may vary with the forms of the preparations. When the form of the preparation is the topical preparation such as an ointment or the like, on the one hand, base components for conventional use with the topical preparations may be employed as the base component. When the form of the preparation is in the form of local preparations, such as suppository or the like, on the other hand, base component compounds which have conventionally been used for suppositories may be employed.

The such base components for the topically adminstrable preparations may include, for example, an ointment base component containing a liquid base component, a liquid agent, an emulsifying base component, and so on, each being capable of solubilizing the pharmaceutically active component compounds and other components.

As the ointment base components, there may be employed, for example, a liquid base component, an organic solid compound, an oily material, a surface active agent, and so on.

As the liquid base components, there may be employed, for example, water acting as a solvent for solubilizing potassium aluminum sulfate, water acting as a solvent for solubilizing tannic acid, ethanol, acetone and so on. When a solution is to be prepared from potassium aluminum sulfate and tannic acid, the solution may be prepared, for example, by mixing a solution obtained by dissolving potassium aluminum sulfate in the solvent such as water or the like containing sodium citrate with a solution obtained separately by dissolving tannic acid in the solvent such as water, ethanol, acetone or the like.

The organic solid compound may include, for example, an aliphatic carboxylic acid such as lauric acid, myristeic acid, palmitic acid, stearic acid, isostearic acid, behenic acid or the like.

Further, the oily material may also be employed as a base component for the present invention. As the oily material, there may be mentioned, for example, an ester of a fatty acid, an ester of an aromatic carboxylic acid, an ester of a phosphoric acid, a triglyceride of a higher fatty acid, a higher aliphatic alcohol, a higher aliphatic acid, terpene, vaseline, lanolin, beeswax, liquid paraffin, squalane, etc., and a mixture thereof.

Furthermore, liquid or solid surface active agents may also be employed. Such surface active agents may include, for example, anionic, ionic, non-ionic and amphoteric surface active agents. It is preferred that non-ionic surface active agents are employed in terms of a low irritation to the skin at the lesions to be applied. Such non-ionic surface active agents may include, for example, a surface active agent of an ethylene oxide type, a surface active agent of a polyhydroxy type, a polymeric type surface active agent, and so on. As the surface active agents of the ethelene oxide type, there may be employed, for example, an ethylene oxide adduct of a higher alcohol, an ethylene oxide adduct of a higher fatty acid, an ethylene oxide adduct of an alkylphenol, an ethylene oxide adduct of an aliphatic amine, an ethylene oxide adduct of an aliphatic amide, an ethylene oxide adduct of a polyvalent alcohol, an ethylene oxide block copolymer, a propylene oxide block copolymer, and so on. As the polyhydroxy surface active agents, there may be employed, for example, a glycerin ester of a mono-fatty acid, a pentaerythritol ester of a fatty acid, a sorbitan ester of a fatty acid, a sucrose ester of a fatty acid, a fatty acid amide of ethanol amine andan alkylene oxide addjut thereof, and so on. For the locally administrable preparations according to the present invention, there may be favorably employed a polyoxyethylene sorbitan ester of a fatty acid, a polyoxyethylene glyceryl ester of a mono-fatty acid, a polyoxypropylene ester of a mono-fatty acid, a sorbitan ester of a fatty acida polyoxyethylene alcohol ether, and so on. These surface active agents may be employed singly or in combination thereof. Further, as solid surface active agents, there may be mentioned, for example, a glycerin ester of a fatty acid, e.g. glyceryl monomyristate, glyceryl monostearate, diglyceryl distearate, etc., a polyglycerin ester of a fatty acid, e.g. tetraglyceryl monostearate, tetraglyceryl tris-stearate, decaglyceryl trioleate, a polyoxyethylene glycerin ester of a fatty acid, e.g. polyoxyethylene (5) glyceryl monostearate, polyoxyethylene (15) glyceryl monostearate, polyoxyethylene (40) glyceryl mono-stearate, a sorbitan ester of a fatty acid, e.g. sorbitan monopalmitate, sorbitan monostearate, sorbitan squistearate, sorbitan tris-stearate, etc., a polyoxyethylene sorbitan, e.g. polyoxyethylene (20) sorbitan tris-stearate, etc., a polyoxyethylene sorbit ester of a fatty acid, e.g. polyoxyethylene (6) sorbit hexastearate, a polyethylene glycol ester of a fatty acid, e.g. polyethylene glycol monostearate (4EO), polyethylene glycol distearate, etc., a polyethylene hardened castor oil, e.g. polyoxyethylene (80) hardened castor oil, polyoxyethylene (100) hardened castor oil, etc., a polyoxyethelene alkyl ether, e.g., polyoxyethylene (2) cetyl ether, polyoxyethylene (5) behenyl ether, etc., a polyoxyethylene phytosterol, e.g. polyoxyethylene (30) phytosterol, etc., a polyoxyethylene phytostanol, e.g. polyoxyethylene (25) phytostanol, etc, a polyoxyethylene polyoxypropylene alkyl ether, e.g., polyoxyethylene (20) polyoxypropylene (8) cetyl ether, polyoxyethylene (20) polyoxypropylene (6) decyl tetradecyl ether, etc., a polyoxyethylene alkyl phenyl ether, e.g., polyoxyethylene (30) octyl phenyl ether, etc., a polyoxyethelene lanolin alcohol, e.g., polyoxyethylene (40) lanolin alcohol, polyoxyethylene (10) lanolin alcohol, etc., a polyoxyethylene beeswax derivative, e.g., polyoxyethylene (6) sorbit beeswax, polyoxyethylene (20) sorbit beeswax, etc., a polyoxyethylene alkyl ether of phosphoric acid, e.g., dipolyoxyethylene (8) alkyl ether of phosphoric acid, etc., and so on.

Furthermore, as the base components for the locally administrable preparations according to the present invention, there may be mentioned, for example, an ester of a fatty acid, an ester of an aromatic carboxylic acid, a triglyceride of a higher fatty acid, an alcohol of a higher fatty acid, and so on.

The esters of the fatty acids may include, for example, an ester of a mono-carboxylic acid and a poly-carboxylic acid in a solid state at ambient temperature. The fatty acid esters may be a higher alcohol ester of a mono- or poly-carboxylic acid, the carboxylic acid being generally saturated or unsaturated, linear or branched-chained and having from 4 to 22 carbon atoms, preferably from 8 to 18 carbon atoms, and the higher alcohol having up to 18 carbon atoms. As the carboxylic acid component of the carboxylic acid ester, on the one hand, there may be mentioned, for example, butyric acid, lactic acid, octanoic acid, isooctanoic acid, dimethyl octanoic acid, nonanoic acid, capric acid, lauric acid, myristic acid, palmitic acid, stearic acid, isostearic acid, oleic acid, behenic acid, adipic acid, azelaic acid, sebacic acid, and so on. As the alcohol component, on the other hand, there may be mentioned, for example, ethanol, propanol, isopropanol, butanol, hexanol, decanol, myristyl alcohol, dodecanol, cetyl alcohol, hexadecyl alcohol, behenyl alcohol, and so on. Therefore, the preferred specific examples of the fatty acid esters may include, for example, a butyric acid ester such as isopropyl butyrate, etc., a lactic acid ester such as myristyl lactate, etc., an octanoic acid ester such as cetyl octanoate, etc., an isooctanoic acid ester such as cetyl isooctanoate, etc., a dimethyloctanoic acid ester such as hexyl decyl dimethyloctanoate, etc., a nonanoic acid ester such as decyl nonanate, etc., a capric acid ester such as isopropyl caprate, etc., a lauric acid ester such as hexyl laurate, etc., a myristic acid ester such as isopropyl myristate, isododecyl myristate, octyl dodecyl myristate, myristyl myristate, etc., a palmitic acid ester such as isopropyl palmitate, etc., a stearic acid ester such as butyl stearate, etc., an isostearic acid ester such as hexyl decyl isostearate, etc., an oleic acid ester such as decyl oleate, oleyl oleate, etc., a behenic acid ester such as behenyl behenate, etc., an adipic acid ester such as oleyl adipate, diethyl adipate, disiobutyl adipate, diisodecyl adipate, dioctyl adipate, dibenzyl adipate, di(2-methoxyethyl) adipate, etc., dimethyl sebacate, an azelaic acid ester such as diisopropyl azelate, diisooctyl zelate, etc., a sebacic acid ester such as diisopropyl sebacate, dioctyl sebacate, etc., or a glycerin or propylene glycol ester of a mono-, di-or tri-fatty acid, such as mono-capric acid glycerin ester, dicapric acid propylene glycol ester, tricapric acid glycerin ester, etc., or the like.

As the aromatic carboxylic acid esters, there may be mentioned, for example, an ester of phthalic acid, e.g. diethyl phthalate, dibutyl phthalate, dioctyl phthalate or the like. An ester of a phosphoric acid may include, for example, triglyceride phosphate, trioleyl phosphate, tridodecyl phosphate, trioctyl phosphate or the like.

Further, in accordance with the present invention, a partial ester of the polyvalent alcohols and an alkylene oxide adduct thereof may also be employed as an oily material.

As the higher aliphatic alcohols, there may be used, for example, cetanol, stearyl alcohol, behenyl alcohol, lanolin alcohol, farnenol or the like. Further, as the higher fatty acids, there may be mentioned, for example, octanoic acid, nonanic acid, capric acid, lauric acid myristic acid, palmitic acid, stearic acid, oleic acid, linoleic acid, linolenic acid, behenic acid, montanic acid, elaidic acid, or the like.

As the higher fatty acid triglycerides, there may be employed various ones which are in a liquid state or in a semi-solid state at ambient temperature and which are naturally occurring animal or plant materials. They are generally referred to as fats and fatty oils and they are commercially available. There may be employed a variety of fats and fatty oils originating from plants and animals such as, for example, animal oils, e.g. beef tallow, liver oil, lanolin, lard or the like. There may be preferably employed vegetable oils such as, for example, olive oil, tsubaki oil, soyabean oil, colza oil, corn oil, castor oil, safflofer oil and so on.

Furthermore, there may be preferably employed ones which are conventionally employed as additives or excipients in Chinese medicine and they may include, for example, seed oils, borneol and so on.

In the locally adminstrable preparations according to the present invention, the amount of the oily material is not limited to a particular one and my appropriately vary with desired kind, property, preparation form of the locally administrable preparations, and so on. Further when the surface active agents are to be employed, the amount thereof is not limited to a particular one and may also vary appropriately with desired kind, property of the locally administrable preparations and so on. When the surface active agent is employed for the locally administrable preparations of a non-emulsion type, the amount of the surface active agent may vary from approximately 5% to 50% by weight, preferably from approximately 20% to 45% by weight, with respect to the total weight of the locally administrable preparation. On the other hand, when the surface active agent is employed for the locally administrable preparations of an emulsion type, the amount of the surface active agent may vary from approximately 1% to 20% by weight, preferably from approximately 5% to 15% by weight, with respect to the total weight of the locally administrable preparation.

The locally administrable preparations according to the present invention may contain, for example, water, fillers, thickening agents (polymer compounds), coloring agents, aromatic agents, emulsion stabilizers, pain lease agent, and so on, as needed, when the preparations are in the form of topical preparations such as ointments or the like. They may be ones which are conventionally employed in the field of the topically administrable preparations, but the those which will be described hereinafter are particularly preferred.

When the locally administrable preparation for treating the affected tissues in accordance with the present invention is employed in the form of an ointment-form mixture of a non-emulsion type, it is preferred that it contains the following composition. In other words, the preferred composition of the ointment-form mixture of a non-emulsion type may preferably comprise, for example, the pharmaceutically active component compounds each in the amount of from approximately 0.1% to 30% by weight, preferably from approximately 1% to 15% by weight, more preferably from approximately 5% to 10% by weight, the oily material in the amount of from approximately 10% to 80% by weight, preferably from approximately 20% to 60% by weight, the surface active agent in the amount of from approximately 20% to 80% by weight, preferably from approximately 40% to 70% by weight, the filler in the amount of approximately 15% by weight, preferably from approximately 5% to 10% by weight, purified water in the amount of approximately 10%, preferably from approximately 1% to 5% by weight.

As the oily materials to be contained in the locally administrable preparations according to the present invention comprising the ointment-form mixture of a non-emulsion type, there may preferably be employed the oily material in a solid state or in a mixed state in which the oily material in a solid state is mixed with the oily material in a liquid state. The locally administrable preparations according to the present invention may contain the oily materials in the amount of from approximately 20% to 80% by weight, preferably from approximately 40% to 70% by weight, with respect to the surface active agent in a solid state at ambient temperature. As the oily materials in the solid state to be used in this case, there may be mentioned, for example, a variety of the surface active agents in the solid form as described hereinabove, but oily materials may also be mentioned.

Further, as the base components to be employed for the local preparations in the form of suppositories or the like, out of the locally administrable preparations for treating the affected tissues in accordance with the present invention, there may also be employed any base materials to be conventionally employed for suppositories. Although the base materials for use with the suppositories may vary with the preparation forms to some extent, there may be mentioned, for example, a solid base material, a hydrophylic base material, a liquid base material, and so on.

As such base materials, there may be employed, for example, an animal-originated fats and oils such as, for example, animal oils such as beef tallow, pig oil, liver oil, egg yolk oil, reduced lard, lard, and so on, including a wax, e.g. beeswax, lanolin, reduced lanolin, and so on; vegetable oils such as olive oil, tsubaki oil, soyabean oil, colza oil, sesame oil, corn oil, peanut oil, peppermint oil, castor oil, safflofer oil, cacao butter, coconut butter, palm oil, palm kernel oil, coconut oil, eucalyptus oil, a higher fatty acid triglyceride including, e.g. witepsol, and so on; a wax, e.g. beeswax, lanolin, reduced lanolin, and so on; a hydrocarbon such as, for example, vaseline, liquid paraffin, squalene, squalane, and so on; an aliphatic carboxylic acid being in a solid state at ambient temperature and having from 10 to 28 carbon atoms, preferably from 10 to 18 carbon atoms, including, for example, a medium- or long-chain aliphatic carboxylic acids such as, for example, capric acid, lauric acid, myristeic acid, palmitic acid, stearic acid, isostearic acid, oleic acid, linoleic acid, linolenic acid, behenic acid, montanic acid, elaidic acid, and so on; a higher alcohol including a higher aliphatic alcohol having from 12 to 22 carbon atoms, such as lauryl alcohol, cetyl alcohol, stearyl alcohol, behenyl alcohol, and so on, and a terpene alcohol including a mono-terbene alcohol, e.g., linalool, etc., and a sesqui-terpene alcohol, e.g., farnenol, etc.; an ester of a fatty acid including, for example, a higher alcohol ester of a monovalent aliphatic carboxylic acid and a polyvalent aliphatic carboxylic acid, wherein each of the monovalent aliphatic carboxylic acid and the polyvalent aliphatic carboxylic acid is in a solid state at ambient temperature and is generally saturated or unsaturated, linear or branched and has from 4 to 22 carbon atoms, preferably from 8 to 18 carbon atoms and wherein the higher alcohol has up to 18 carbon atoms, i.e. the carboxylic acid component of the fatty acid ester including, for example, butyric acid, lactic acid, octanoic acid, isooctanoic acid, dimethyloctanoic acid, nonanoic acid, capric acid, lauric acid, myristic acid, palmitic acid, stearic acid, isostearic acid, oleic acid, behenic acid, adipic acid, azelaic acid, sebacic acid, and so on, and the alcohol component thereof including, for example, ethanol, propanol, isopropanol, butanol, hexanol, decanol, myristyl alcohol, dodecanol, cetyl alcohol, hexadecyl alcohol, behenyl alcohol, and so on, that is, the aliphatic acid ester including, for example, a butyric acid ester e.g. isopropyl butyrate, etc., a lactic acid ester, e.g. myristyl lactate, etc., an octanoic acid ester, e.g. cetyl octanoate, etc., an isooctanoic acid ester, e.g. cetyl isooctanoate, etc., a dimethyloctanoic acid ester, e.g. hexyl decyl dimethyloctanoate, etc., a nonanoic acid ester, e.g. decyl nonanate, etc., a capric acid ester, e.g. isopropyl caprate, etc., a lauric acid ester, e.g. hexyl laurate, etc., a myristic acid ester, e.g. isopropyl myristate, isododecyl myristate, octyl dodecyl myristate, myristyl myristate, etc., a palmitic acid ester, e.g. isopropyl palmitate, etc., a stearic acid ester, e.g. butyl stearate, etc., an isostearic acid ester, e.g. hexyl decyl isostearate, etc., an oleic acid ester, e.g. decyl oleate, oleyl oleate, etc., a behenic acid ester, e.g. behenyl behenate, etc., an adipic acid ester, e.g. oleyl adipate, diethyl adipate, diisobutyl adipate, diisodecyl adipate, dioctyl adipate, dibenzyl adipate, di(2-methoxyethyl) adipate, etc., dimethyl sebacate, an azelaic acid ester, e.g. diisopropyl azelate, diisooctyl azelate, etc., a sebacic acid ester, e.g. diisopropyl sebacate, dioctyl sebacate, etc.; an ester of a medium- or long-chain carboxylic acid such as, for example, triolefin, tristearin and so on; an ester of an aromatic carboxylic acid including a phthalic acid ester such as, for example, diethyl phthalate, dibutyl phthalate, dioctyl phthalate, and so on; an ester of a phosphoric acid such as, for example, trioleyl phosphate, tridodecyl phosphate, trioctyl phosphate, and so on; and the oily materials as described hereinabove, such as, for example, a partial ester of the polyvalent alcohols and an alkylene oxide adducts thereof.

The locally administrable preparations in the form of suppositories in accordance with the present invention may contain, as needed, a surface active agent, excipient, filler, thickening agent (polymer compound), pain releasing agent, preservative, coloring agent, aromatic agent, and other agents. Each or many of those components may also be used in common with the locally administrable preparations in the forms of the topical preparations to be used for the present invention.

For the locally administrable preparations for treating the affected tissues in accordance with the present invention, which are in the form of suppositories, the surface active agents to be employed therefor, as needed, may include anionic, ionic, non-ionic and ampholytic. Out of them, the non-ionic surface active agent is preferred from the pointof view that irritation is low to the skin at the lesions to which the preparations are applied. Those surface active agents as described hereinabove may also be employed as the surface active agents for the suppository form of the preparations. Particularly, for the suppository form of the locally administrable preparations according to the present invention, there may be employed, for example, the polyoxy-ethylene sorbitan ester of the fatty acid, the polyoxyethylene glyceryl ester of the monovalent fatty acid, the polyoxypropylene ester of the monovalent fatty acid, the sorbitan ester of the fatty acidthe polyoxyethylene alcohol ether, and so on. Further, the surface active agents in the solid state may also be employed and those solid surface active agents as described hereinabove may be used as the surface active agents in the solid state to be employed in this case.

As the excipients, there may be employed any excipient which are usually employed for medicinal preparations and such excipients may include, for example, dextran, glycerin and so on. Further, additives or excipients which are conventionally employed extensively in Chinese medicine may also be used, and they may include, for example, seed oil, borneol, and so on.

Further, as the fillers, there maybe used organic and inorganic fine powders such as, for example, zinc oxide, silica, alumina, titania, resinous powders, silicate powders, clay powders, sepiolite powders, montmorillonite powders, fluorine-containing mica powders, hydroxypropyl cellulose powders, and so on. They may usually have an average particle size in the range of from approximately 0.1 µm to 20 µm, preferably from approximately 0.5 µm to 10 µm.

Moreover, the thickening agents may be employed, which may include, for example, a water-soluble polymer compound such as, e.g. carboxyvinyl polymer, methylcellulose, hydroxyethyl cellulose, carboxymethyl cellulose, sodium alginate, alginic acid propylene glycol ester, chitosan, polyvinyl alcohol, sodium starch glycollate, and so on.

Furthermore, as the coloring agents and the aromatic agents for the locally administrable preparations according to the present invention, there may be employed any one that is conventionally employed for the locally administrable preparations in the form of the local or topical preparations such as ointments, suppositories, etc.

In addition, the locally administrable preparations may contain a variety of medicinal substances although they vary with indications for the use of the medicine. Such medicinal substances may include, for example, a local anesthetic, an antibiotic, an antibacterial or antifungal substance, a steroidal anti-inflammatory substance, a non-steroidal anti-inflammatory substance, or the like. As the local anesthetics as a pain releasing agent, there may be mentioned, for example, lidocaine, lidocaine hydrochloride, procaine, dibucaine, tetracaine, and so on. As the antibiotics, there may be mentioned, for example, penicillin, erythromycin, tetracycline, chloramphenicol, fradiomycin hydrochloride, and so on. The antibacterial and antifungal substances may include, for example, nitrofurazone, nystatin, pyrrolnitrin, clotrimazole, miconazole, isoconazole, econazole, trifonazole, tolnaftate, and so on. As the steroidal antiinflammatory substance, there may be mentioned, for example, prednisolone, paramethasone, flumethasone, dexamethasone, betamethasone valerate, fluocinonide, fluocinolone acetonide, hydrocortisone acetate, clobetasol propionate, hydrocortisone, triamcinolone, triamcinolone acetonide, and so on. The non-steroidal anti-inflammatory substances, there may be mentioned, for example, methyl salicylate, glycol salicylate, acetyl salicylate, indomethacin, ibuprofen, flurbiprofen, naproxen, piroxicam, diclofenac, alclofenac, oxyphenbutazone, phenylbutazone, and so on.

The locally administrable preparations according to the present invention may be prepared, for example, by mixing a solution of the pharmaceutically active component compounds with a molten mixture of the molten oily material and surface active agent and then, as needed, adding the filler to the resulting mixture, mixing the mixture uniformly, and allowing the uniform mixture to cool.

When the locally administrable preparation for treating the affected tissues in accordance with the present invention is applied in the form of the ointment-form mixture of an emulsion type, it is preferred that it has the composition which may contain, for example, each of the pharmaceutically active component compounds at the rate of from approximately 0.1% to 30% by weight, preferably from approximately 1% to 15% by weight, more preferably from approximately 5% to 10% by weight, the oily materials at the rate of from approximately 60% to 90% by weight, preferably from approximately 75% to 85% by weight, the surface active agent at the rate of from approximately 1% to 20% by weight, preferably from approximately 2% to 10% by weight, the filler at the rate up to approximately 15%, preferably from approximately 5% to 10% by weight, purified water at the rate up to approximately 10% by weight, preferably from approximately 1% to 5% by weight, each on the basis of the total weight of the locally administrable preparation.

For the locally administrable preparations according to the present invention, the oily materials to be employed may be in a solid state at ambient temperature or a mixture of the oily material in a solid state with one in a liquid state at ambient temperature. Further, the surface active agents may preferably be employed which have a HLB value of from 8 to 15, preferably from 9 to 12.

The locally administrable preparations according to the present invention may be prepared by preparing each of the pharmaceutically active component compounds or a solution thereof and the oily material and/or the surface active agent separately and mixing them as needed. For example, each of the pharmaceutically active component compounds or the solution thereof is warmed to about 60 °C or higher, and the resulting material is in turn added gradually to a molten solution of the oily material and/or the surface active agent with stirring. To the resulting mixture, the filler is added as needed and the resulting mixture is allowed to cool, thereby yielding the locally administrable preparations.

When the locally administrable preparations for treating the affected tissues in accordance with the present invention are applied in the state of a cream-form mixture of an emulsion type, they may preferably comprise the composition which may contain, for example, each of the pharmaceutically active component compounds at the rate of from approximately 0.1% to 20% by weight, preferably from approximately 3% to 10% by weight, the organic solvent at the rate of from approximately 1% to 40% by weight, preferably from approximately 2% to 20% by weight, the oily material at the rate of from approximately 2% to 50% by weight, preferably from approximately 10% to 40% by weight, the surface active agent at the rate of from approximately 10% to 35% by weight, preferably from approximately 15% to 30% by weight, the thickening agent (water-soluble polymer) at the rate of from approximately 0.1% to 5% by weight, preferably from approximately 0.2% to 2% by weight, purified water at the rate of from approximately 30% to 75% by weight, preferably from approximately 40% to 60% by weight, the filler at the rate up to approximately 10% by weight, preferably at the rate of from approximately 1% to 5% by weight, each on the basis of the weight of the preparation.

More specifically, in preparing the locally administrable preparations, an aqueous solution may be prepared by mixing the pharmaceutically active component compounds in purified water or the organic solvent and warming the resulting mixture. On the other hand, a molten mixture may be prepared by mixing the oily materials with the surface active agents while being warmed, and purified water is added gradually to the resulting molten mixture. To the resulting molten mixture was gradually added the solution of the pharmaceutically active component compounds, separately prepared, with stirring while being warmed, and the thickening agent is added to the resulting mixture. To the resulting mixture is further added the filler as needed. By preparing the locally administrable preparations in the manner as described hereinabove, there may be prepared the locally administrable preparations in an emulsion of an oil/water type and a water/oil type. When the emulsion of an oil/water type is to be prepared, it is preferred to use the surface active agent having an HLB value in the range of 9 to l8. On the other hand, when the emulsion of a water/oil type is to be prepared, it is preferred to use the surface active agent having an HLB value in the range of 2 to 8. The oily materials to be employed in this case may be ones in a solid state at ambient temperature or a mixture of the solid ones with ones in a liquid state at ambient temperature. The thickening agent to be used therein may include the water-soluble polymers such as, for example, carboxyvinyl polymer, methylcellulose, hydroxyethyl cellulose, carboxymethyl cellulose, sodium alginate, propylene glycol alginate, chitosan, polyvinyl alcohol, sodium starch glycollate, and so on.

The preferred method for preparing the locally administrable preparations in a cream form in accordance with the present invention may comprise, for example, dissolving the pharmaceutically active component compounds in purified water or the organic solvent, adding to the resulting solution a molten mixture prepared by mixing the solid oily substance and the solid surfactant and melting the mixture, and further adding a solution of the pharmaceutically active component compounds to the resulting mixture. In the mixing step, it is preferred, for instance, to set the temperature for melting the oily materials in the solid state at ambient temperature and the surface active agents in the solid state at ambient temperature to the temperature that can be maintained higher than the melting point of each of the materials yet to cause no precipitating.

The resulting mixture is then mixed with water and the thickening agent, and the filler is added to the resulting mixture, as needed, followed by allowing the resulting mixture to cool. In this case, it is preferred to add the thickening agent after the addition of water. It is to be noted, however, that the method for preparing the locally adminstrable preparations is not limited to this method. It is also possible to dissolve the thickening agent and the filler to be added as needed in water and to add the resulting aqueous solution thereto. For instance, the water to be employed may contain a water-soluble substance such as, for example, urea or a polyvalent alcohol as described hereinabove. The amount of urea to be added in this case may be in the rate of from approximately 5% to 20% by weight, preferably from approximately 5% to 10% by weight. Further, the water may be preferably adjusted in advance to pH4.5 to pH5.5 with, for example, a phosphate buffer solution or the like.

The locally administrable preparations in the form of a mixture in a cream state prepared in the manner as described hereinabove can be prepared as a mixture in which each of their ingredients is mixed together in a uniform manner for instance, by adding two portions of the aqueous solution of each of the pharmaceutically active component compounds in water or the solution thereof in the organic solvent to the molten mixture of the oily materials and the surface active agents in such a manner that the first portion thereof is added without stirring or with slowed stirring and that the second portion thereof is added with rapid stirring, thereby enabling each of the components in the resulting mixture to be mixed uniformly. The resulting mixture can be formulated to provide a preparation having a remarkably high degree of stability.

When the locally administrable preparation for treating the affected tissues in accordance with the present invention are applied in the form of a lotion of a solution type, they may preferably have the following composition which may preferably contain, for example, the pharmaceutically active component compounds at the rate of from approximately 0.1% to 20% by weight, preferably from approximately 3% to 10% by weight, the organic solvent at the rate of from approximately 2% to 40% by weight, preferably from 10% to 30% by weight, the oily material in a liquid state at the rate up to approximately 30% by weight, preferably up to approximately 20% by weight, the surface active agent at the range up to approximately 20% by weight, preferably up to approximately 7% by weight, water at the range up to 80% by weight, preferably up to approximately 60% by weight, the thickening agent at the rate of from approximately 0.05% to 5% by weight, preferably from approximately 0.2% to 1% by weight, with respect to the weight of the preparation.

The locally administrable preparations according to the present invention as stated hereinabove may be prepared by dissolving the pharmaceutically active component compounds in the organic liquid to yield a solution and adding appropriately to the resulting solution the oily materials in a liquid state, the surface active agents, the solvents and/or the thickening agents. The resulting preparations in the lotion type may be applied as they are as a liquid lotion of the preparations. For instance, they may be applied in an aerosol type by filling the resulting preparations in an aerosol can together with an atomizing agent such as ka natural gases.

The locally administrable preparations according to the present invention may be administered by applying them, for example, from one to several times, e.g. three times, per day, directly to the lesions in accordance with the preparation forms. It is to be noted, however, that the times of application can be appropriately increased or decreased in accordance with the extent of the symptoms and the preparation forms.

As the locally administrable preparations according to the present invention contain the pharmaceutically active component compounds each having a high degree of safety and each is dissolved in the organic liquid or the organic solid material, the locally administrable preparations according to the present invention cause no inflammation and can be absorbed at a remarkably high absorption rate through the skin or the mucous membrane, thereby achieving a high level of treatment effects.

### BEST MODES FOR CARRYING OUT THE INVENTION

### Examples

The present invention will be described in more detail by way of examples.

### Example 1

A mixture was prepared by mixing 100 grams of tannic acid and 20 grams of sodium hydrogen sulfite with 100 ml of glycerin, and the resulting mixture was heated and ground in a mortar into powders.

Separately, 20 grams of potassium aluminum sulfate, 20 grams of lidocaine, and 20 grams of borneol were ground in a mortar into powders and screened to yield powders having predetermined particle sizes.

The two kinds of the powders were combined together and mixed to make them uniform and the mixture was molten. To the uniform mixture was added vaseline to make the total amount 1,000 grams, thereby yielding an ointment. The resulting ointment was then filled in tubes for storage.

### Example 2

A mixture was prepared by mixing 100 grams of tannic acid and 20 grams of sodium hydrogen sulfite with 10 ml of glycerin, and the resulting mixture was heated and ground in a mortar into powders.

Separately, 20 grams of potassium aluminum sulfate, 20 grams of procaine, and 20 grams of borneol were ground in a mortar into powders and screened to yield powders having predetermined particle sizes.

The two kinds of the powders were combined together and mixed to make them uniform, followed by melting the mixture and adding vaseline to the uniform mixture so as to make the total amount of the resulting mixture 1,000 grams, thereby yielding an ointment. The resulting ointment was then filled in tubes.

### Example 3

A mixture was prepared by mixing 100 grams of tannic acid and 20 grams of sodium hydrogen sulfite with 100 ml of glycerin, and the resulting mixture was heated and ground in a mortar into powders.

Separately, 20 grams of potassium aluminum sulfate, 1 gram of penicillin, and 20 grams of borneol were ground in a mortar into powders and screened to yield powders having predetermined particle sizes.

The two kinds of the powders were combined together and mixed uniformly. After the mixture was molten, the resulting molten uniform mixture was added with vaseline to make the total amount thereof 1,000 grams, thereby yielding an ointment. The resulting ointment was then filled in tubes.

### Example 4

A mixture was prepared by mixing 100 grams of tannic acid and 20 grams of sodium hydrogen sulfite with 100 ml of glycerin, and the resulting mixture was heated and ground in a mortar into powders.

Separately, 20 grams of potassium aluminum sulfate, 20 grams of prednisolone, and 20 grams of borneol were ground in a mortar into powders and screened to yield powders having predetermined particle sizes.

The two kinds of the powders were combined together and mixed to make them uniform, followed by melting the mixture by heating and then adding vaseline to the uniform mixture so as to make the total amount thereof 1,000 grams, thereby yielding an ointment. The resulting ointment was then filled in tubes.

### Example 5

A mixture was prepared by mixing 100 grams of tannic acid and 20 grams of sodium hydrogen sulfite with 100 ml of glycerin, and the resulting mixture was heated and then ground in a mortar into powders.

Separately, 20 grams of potassium aluminum sulfate, 10 grams of ibuprofen and 20 grams of borneol were ground in a mortar into powders and filtered to collect the powders having predetermined particle sizes.

The two kinds of the powders were then combined together and mixed uniformly. After the mixture was molten by heating, vaseline was added to the uniform mixture so as to make the total amount thereof 1,000 grams, thereby yielding an ointment. The resulting ointment was then filled in tubes.

### Example 6

A mixture was prepared by mixing 100 grams of tannic acid and 20 grams of sodium hydrogen sulfite with 100 ml of glycerin, and the resulting mixture was heated and ground in a mortar into powders.

Separately, 20 grams of potassium aluminum sulfate and 30 grams of borneol were ground in a mortar into powders and filtered to yield the powders having the predetermined particle size.

The two kinds of the powders were combined together and mixed to make them uniform and the mixture was molten, followed by adding vaseline to the uniform mixture so as to make the total amount thereof 1,000 grams, thereby yielding an ointment. The resulting ointment was then filled in tubes.

### Example 7

A mixture was prepared by mixing 100 grams of tannic acid and 20 grams of sodium hydrogen sulfite with 100 ml of glycerin, and the resulting mixture was heated and ground in a mortar into powders. Likewise, 20 grams of potassium aluminum sulfate, 20 grams of lidocaine, and 20 grams of borneol were ground in a mortar into powders and filtered to yield the powders having the predetermined particle size. The two kinds of the powders were combined together and mixed uniformly.

To the resulting powdery mixture were added 30 grams of behenyl alcohol, 30 grams of propylene carbonate, 100 grams of olive oil, 10 grams of dibutyl adipate, 30 grams of isopropyl myristeate, 30 grams of isotridecyl myristeate, 40 grams of cetyl palmitate, 30 grams of stearic acid, 30 grams of polyoxyethylene (5) glyceryl stearate, 30 grams of polyethylene glycol monostearate, and 10 grams of poly-oxyethylene (2) cetyl alcohol. The resulting mixture was molten at 85°C or higher to yield a uniform solution.

Further, a mixture consisting of 20 grams of diisopropanol amine, 50 grams of isoprene glycol, 1 gram of propyl p-aminobenzoate and 1 gram of methyl p-aminobenzoate was added to approximately 450 ml of purified water, and the resulting mixture was warmed to 80°C to yield a uniform dispersion. While the resulting uniform dispersion was rapidly stirred at 80°C, it was gradually added to the solution separately prepared to thereby yield an emulsion. After the addition of the mixture was completed, the warming was suspended and the resulting emulsion was allowed to cool to 60°C to 55°C while stirring at room temperature. Thereafter, purified water was added to make the entire weight of the emulsion 1 kilogram. The resulting composition was allowed to stand at room temperature to remove foam, followed by filling the resulting composition into containers for cream.

### Example 8:

A mixture was prepared by mixing 100 grams of tannic acid and 20 grams of sodium hydrogen sulfite with 100 ml of glycerin, and the resulting mixture was heated and ground in a mortar into powders. Likewise, 20 grams of potassium aluminum sulfate, 20 grams of lidocaine, and 20 grams of borneol were ground in a mortar into powders and filtered to yield the powders having a predetermined range of the particle sizes. The two kinds of the powders were combined together and mixed yielding a uniform mixture.

To the resulting powdery mixture was added a mixture consisting of 100 grams of olive oil, 50 grams of whale wax, 30 grams of behenyl alcohol, 30 grams of isopropyl myristate, 30 grams of isotridecyl myristate, 30 grams of polyoxyethylene (5) glyceryl stearate, 30 grams of polyethylene glycol monostearate, and 10 grams of polyoxyethylene (2) cetyl alcohol monostearate. The resulting mixture was heated to 82°C or higher and dissolved yielding a uniform solution.

Further, a mixed solution was prepared by admixing 20 grams of diisopropanol amine, 30 grams of stearic acid, 1 gram of propyl p-aminobenzoate, 1 gram of methyl p-aminobenzoate and 50 grams of isoprene glycol, and the mixed solution was added to approximately 450 ml of purified water, followed by warming the resulting mixture to 80°C to yield a uniform dispersion. While the resulting uniform dispersion was rapidly stirred at 80°C, the solution, separately prepared, was gradually added to the resulting dispersion to thereby yield an emulsion. After the addition of the solution was completed, the warming was suspended and the resulting emulsion was allowed to cool to 60°C to 55°C while stirring at room temperature. Thereafter, purified water was added to the emulsion to make the entire weight thereof 1 kilogram. The resulting composition was allowed to stand at room temperature and defoamed to yield cream and the resulting cream was then filled in containers for cream.

### Example 9:

A mixture was prepared by mixing 50 grams of tannic acid and 50 grams of sodium hydrogen sulfite with 100 ml of glycerin, and the resulting mixture was heated and ground in a mortar into powders. Likewise, 20 grams of potassium aluminum sulfate, 20 grams of lidocaine, and 200 grams of vaseline were ground in a mortar into powders and filtered to yield the powders having a predetermined range of the particle sizes. The two kinds of the powders were combined together and mixed to yield a uniform mixture.

To the resulting powdery mixture was added a mixture consisting of 100 grams of olive oil, 30 grams of behenyl alcohol, 30 grams of propylene carbonate, 10 grams of dibutyl adipate, 30 grams of isopropyl myristeate, 30 grams of isotridecyl myristeate, 40 grams of cetyl palmitate, 30 grams of stearic acid, 30 grams of polyoxyethylene (5) glyceryl stearate, 30 grams of polyethylene glycol monostearate, and 10 grams of polyoxyethylene (2) cetyl alcohol monostearate. The resulting mixture was then heated at 82°C or higher and dissolved to yield a solution.

Separately, a mixture was prepared by admixing 20 grams of diisopropanol amine, 50 grams of inoprene glycol, 1 gram of propyl p-aminobenzoate, and 1 gram of methyl p-aminobenzoate, and the resulting mixture was added to approximately 450 ml of purified water, followed by warming the resulting mixture to 80°C yielding a uniform dispersion. While the resulting uniform dispersion was maintained at 80°C, the resulting solution was gradually added to the resulting uniform dispersion with rapidly stirring, to thereby yield an emulsion. After the emulsification was completed, the warming was suspended and the resulting emulsion was allowed to cool to 60°C to 55°C while stirring at room temperature. Thereafter purified water was added to the emulsion so as to make the entire weight thereof 1 kilogram. The resulting composition was allowed to stand at room temperature to remove foam to yield cream and the resulting cream was then filled in containers for cream.

### Example 10:

A mixture was prepared by mixing 200 grams of tannic acid and 70 grams of sodium hydrogen sulfite with 200 ml of glycerin, and the resulting mixture was heated and ground in a mortar into powders. Likewise, 30 grams of potassium aluminum sulfate, 20 grams of lidocaine, and 200 grams of vaseline were ground in a mortar into powders which in turn were filtered to yield the powders having a predetermined range of the particle sizes. The two kinds of the powders were combined together and mixed yielding a uniform mixture.

To the resulting powdery mixture was added a mixture consisting of 40 grams of cetanol, 50 grams of squalene, 50 grams of polyethylene glycol-400, 60 grams of isoprobyl myristate, 120 grams of polyoxyethylene (5) glyceryl stearate, 20 grams of sorbitan monostearate, and 20 grams of polyoxyethylene (2) cetyl alcohol. The resulting mixture was heated at 82°C or higher and dissolved yielding a uniform solution which in turn was maintained at the same temperature.

Separately, a mixture consisting of 30 grams of propylene glycol, 20 grams of butylene glycol, 20 grams of diisopropanol amine, 10 grams of carboxyvinyl polymer, 1 gram of methyl p-aminobenzoate, 1 gram of propyl p-aminobenzoate and 20 grams of tetracycline hydrochloride was added to approximately 400 ml of purified water, and the resulting mixture was warmed to 80°C and dissolved to yield a solution.

To the resulting solution maintained at 80°C or higher, the solution separately prepared was gradually added, while rapidly stirring, to thereby yield a water-in-oil type emulsion. After the addition of the solution was completed, the warming was suspended and the resulting emulsion was allowed to cool to 60°C to 55°C while stirring at room temperature. Thereafter, purified water was added to make the entire weight 1 kilogram, and the resulting composition was allowed to stand at room temperature and defoamed to yield cream, followed by filling the resulting cream into containers for cream

### Example 11:

A mixture was prepared by mixing 100 grams of tannic acid and 20 grams of sodium hydrogen sulfite with 100 ml of glycerin, and the resulting mixture was heated and ground in a mortar into powders. Likewise, 20 grams of potassium aluminum sulfate, 20 grams of lidocaine, and 20 grams of borneol were ground in a mortar into powders which in turn were filtered to yield the powders having a predetermined range of the particle sizes. The two kinds of the powders were combined together and mixed yielding a uniform mixture.

To the resulting powdery mixture was added a mixture consisting of 30 grams of behenyl alcohol, 30 grams of stearic acid, 60 grams of isostearic acid, 20 grams of isopropyl myristate, 20 grams of isotridecyl myristate, 30 grams of cetyl palmitate, 30 grams of polyoxyethylene (5) glyceryl stearate, 30 grams of polyethylene glycol monostearate, 20 grams of polyoxyethylene (2) cetyl alcohol ether, and 80 grams of olive oil. The resulting mixture was heated at 82°C or higher and dissolved to yield a uniform solution.

Separately, a mixture consisting of 20 grams of diisopropanol amine, 50 grams of isoprene glycol, 1 gram of propyl p-aminobenzoate, and 1 gram of methyl p-aminobenzoate was added to approximately 450 ml of purified water and the resulting mixture was warmed to 80°C and dissolved yielding a uniform dispersion.

To the resulting dispersion maintained at 80°C, the solution separately prepared was gradually added, while rapidly stirring, to thereby yield an emulsion. After the addition of the solution was completed, the warming was suspended and the resulting emulsion was allowed to cool to 60°C to 55°C while stirring at room temperature. Thereafter, purified water was added to the emulsion so as to make the entire weight thereof 1 kilogram, and the resulting composition was allowed to stand at room temperature and defoamed to yield cream, followed by filling the resulting cream into containers for cream.

### Example 12:

A mixture was prepared by mixing 200 grams of tannic acid and 70 grams of sodium hydrogen sulfite with 200 ml of glycerin, and the resulting mixture was heated and ground in a mortar into powders. Likewise, 30 grams of potassium aluminum sulfate, 20 grams of lidocaine, and 200 grams of vaseline were ground in a mortar into powders and filtered to yield the powders having a predetermined range of the particle sizes. The two kinds of the powders were combined together and dissolved yielding a uniform mixture.

To the resulting powdery mixture was added a mixture consisting of 10 grams of propylene carbonate, 40 grams of cetanol, 60 grams of squalene, 60 grams of isopropyl myristate, 30 grams of polyoxyethylene (5) glyceryl stearate, 30 grams of polyethylene glycol monostearate (40EO), and 20 grams of sorbitan monostearate. The caught mixture was heated at 82°C or higher and dissolved yielding a uniform solution which in turn was stored at the same temperature.

Separately, a mixture consisting of 20 grams of diisopropanol amine, 50 grams of propylesi glycol, 10 grams of carboxyvinyl polymer, 1 gram of probyl p-aminobenzoate, and 1 gram of methyl p-aminobenzoate was added to approximately 450 ml of purified water, and the resulting mixture was warmed to 80°C and dissolved yielding a uniform solution.

Then, the resulting uniform solution was gradually added to the separately prepared solution maintained at 80°C, while rapidly stirring, to thereby yield an emulsion. After the addition of the solution was completed, the warming was suspended and the resulting emulsion was allowed to cool to 60°C to 55°C while stirring at room temperature Thereafter, purified water was added to make the total weight thereof 1 kilogram, and the resulting composition was allowed to stand at room temperature and defoamed to yield cream, followed by filling the resulting cream into containers for cream.

### Example 13:

A mixture was prepared by mixing 50 grams of tannic acid and 50 grams of sodium hydrogen sulfite with 100 ml of glycerin, and the resulting mixture was heated and ground in a mortar into powders. Likewise, 20 grams of potassium aluminum sulfate, 20 grams of lidocaine, and 200 grams of vaseline were ground in a mortar into powders and filtered to yield the powders having a predetermined range of the particle sizes, The two kinds of the powders were combined together and mixed uniformly.

To the resulting powdery mixture was added a mixture consisting of 50 grams of stearyl alcohol, 300 grams of white vaseline, 40 grams of polyoxyethylene hardened castor oil, and 10 grams of glycerin monostearate was heated at 83°C and dissolved yielding a uniform solution.

Further, a mixture consisting of 120 grams of propylene glycol, 1 gram of methyl p-aminobenzoate, and 1 gram of propyl p-aminobenzoate was added to approximately 350 ml of purified water, and the resulting mixture was warmed to 80°C and dissolved yielding a solution. The resulting solution was gradually added to the separately prepared solution while being maintained at 80°C and rapidly stirred, to thereby yield a water-in-oil type emulsion. After the addition of the solution was completed, the warming was suspended and the resulting emulsion was allowed to cool to 60°C to 55°C while stirring at room temperature. Thereafter, purified water was added to make the total weight 1 kilogram, and the resulting composition was allowed to stand at room temperature and defoamed to yield cream, followed by filling the resulting cream into containers for cream.

### Example 14:

A mixture was prepared by mixing 50 grams of tannic acid and 50 grams of sodium hydrogen sulfite with 100 ml of glycerin, and the resulting mixture was heated and ground in a mortar into powders. Likewise, 20 grams of potassium aluminum sulfate, 20 grams of lidocaine, and 200 grams of vaseline were ground in a mortar into powders and filtered to yield the powders having a predetermined range of the particle sizes. The two kinds of the powders were combined together and mixed uniformly.

To the resulting powdery mixture was added a mixture consisting of 30 grams of behenyl alcohol, 30 grams of propylene carbonate, 100 grams of olive oil, 10 grams of dibutyl adipate, 30 grams of inopropyl myristate, 30 grams of isotridecyl myristeate, 40 grams of cetyl palmitate, 30 grams of stearic acid, 30 grams of polyoxyethylene (5) glyceryl stearate, 30 grams of polyethylene glycol monostearate, and 10 grams of polyoxyethylene (2) cetyl alcohol. The resulting mixture was heated at 82°C or higher and dissolved yielding a uniform solution.

Separately, 20 grams of diisopropanol amine, 50 grams of isoprene glycol, 1 gram of methyl p-aminobenzoate, and 1 gram of propyl p-aminobenzoate were mixed together uniformly and added to approximately 450 ml of purified water, followed by warming the resulting mixture to 80°C to yield a uniform dispersion. While the uniform dispersion was continued warming at 80°C, the solution prepared separately was gradually added thereto with rapidly stirring to yield an emulsion. After the addition of the solution was completed, the warming was suspended and the resulting mixture was allowed to cool to 60°C to 55°C while stirring at room temperature. Thereafter, purified water was added at 60°C to 55°C to make the total weight of the emulsion 1 kilogram. The resulting emulsion was allowed to stand at room temperature and defoamed to cream, followed by filling the resulting composition into containers for cream.

### Example 15:

A mixture was prepared by mixing 100 grams of tannic acid and 20 grams of sodium hydrogen sulfite with 100 ml of glycerin, and the resulting mixture was heated and ground in a mortar into powders. Likewise, 20 grams of potassium aluminum sulfate, 20 grams of lidocaine, and 20 grams of borneol were ground in a mortar into powders and filtered to yield the powders having a predetermined range of the particle sizes. The two kinds of the powders were combined together and mixed yielding a uniform mixture.

To the resulting powdery mixture was added a mixture consisting of 30 grams of behenyl alcohol, 100 grams of olive oil, 50 grams of whale wax, 30 grams of isopropyl myristate, 30 grams of isotridecyl myristate, 30 grams of polyoxyethylene (5) glyceryl stearate, 30 grams of polyethylene glycol monostearate, and 10 grams of polyoxyethylene (2) cetyl alcohol. The resulting mixture was mixed uniformly and heated at 82°C or higher, thereby dissolving them to yield a uniform solution.

Separately, 20 grams of diisopropanol amine, 50 grams of isoprene glycol, 30 grams of stearic acid, 1 gram of propyl p-aminobenzoate, and 1 gram of methyl p-aminobenzoate were mixed together uniformly and added to approximately 450 ml of purified water, followed by warming the resulting mixture to 80°C and yielding a uniform dispersion.

While the uniform dispersion was continued warming at 80°C, the solution prepared separately was gradually added thereto with rapidly stirring to yield an emulsion. After the addition of the solution was completed, the warming was suspended and the resulting emulsion was allowed to cool to 60°C to 55°C while stirring at room temperature. Thereafter, purified water was added to the resulting emulsion to make the total weight 1 kilogram. The resulting emulsion was allowed to stand at room temperature and defoamed to yield cream, followed by filling the resulting composition into containers for cream.

### Example 16:

A mixture was prepared by mixing 150 grams of tannic acid and 80 grams of sodium hydrogen sulfite with 200 ml of glycerin, and the resulting mixture was heated and ground in a mortar into powders. Likewise, 50 grams of potassium aluminum sulfate, 30 grams of lidocaine, and 300 grams of vaseline were ground in a mortar into powders and filtered to yield the powders having a predetermined range of the particle sizes. The two kinds of the powders were combined together and mixed uniformly.

To the resulting powdery mixture was added a mixture consisting of 80 grams of olive oil, 30 grams of behenyl alcohol, 10 grams of polyethylene glycol (molecular weight: 400), 30 grams of isopropyl myristate, 30 grams of isotridecyl myristate, 70 grams of cetyl palmitate, 30 grams of polyoxyethylene (5) glyceryl stearate, 20 grams of polyethylene glycol monostearate, and 20 grams of sorbitan monostearate. The resulting mixture was heated at 82°C or higher and dissolved yielding a uniform solution.

Separately, 20 grams of diisopropanol amine, 20 grams of butylene glycol, 30 grams of isoprene glycol, 5 grams of carboxyvinyl polymer, 40 grams of stearic acid, 1 gram of propyl p-aminobenzoate, and 1 gram of methyl p-aminobenzoate were mixed together and added to approximately 500 ml of purified water, followed by warming the resulting mixture to 80°C to yield a uniform dispersion.

While the uniform dispersion was maintained at 80°C, the solution prepared separately was gradually added thereto with rapidly stirring to yield an emulsion. After the addition of the solution was completed, the warming was suspended and the resulting emulsion was allowed to cool to 60°C to 55°C while stirring at room temperature. Thereafter, purified water was added to the resulting emulsion to make the total weight of the emulsion 1 kilogram. The resulting emulsion was then allowed to stand at room temperature and defoamed to yield cream, followed by filling the resulting composition into containers for cream.

### Example 17:

A mixture was prepared by mixing 50 grams of tannic acid and 50 grams of sodium hydrogen sulfite with 100 ml of glycerin, and the resulting mixture was heated and ground in a mortar into powders. Likewise, 20 grams of potassium aluminum sulfate, 20 grams of lidocaine, and 200 grams of vaseline were ground in a mortar into powders and filtered to yield the powders having a predetermined range of the particle sizes. The two kinds of the powders were combined together and mixed to make them uniform.

Separately, 15 grams of white vaseline, 5 grams of cetanol, 5 grams of stearyl alcohol, 3 grams of squalane, 2 grams of polyoxyethylene cetyl alcohol and 0.2 gram of methylbarabene were mixed together and heated to 80°C and the resulting mixture was dissolved in the above solution with uniformly stirring. To the resulting solution was added 55 grams of purified water warmed to the same temperature, and the resulting mixture was stirred with a homogenizer to yield an emulsion. To the resulting emulsion was then added a solution containing the uniform mixture of the pharmaceutically active component compounds prepared in the manner as described hereinabove while warming at approximately 45°C, thereby yielding a cream preparation.

### Example 18:

A cream base was prepared from 15 grams of white vaseline, 5 grams of cetanol, 5 grams of stearyl alcohol, 2 grams of polyoxyethylene cetyl alcohol, and 2 grams of liquid paraffin in substantially the same manner as in Example 17. To the resulting cream base was added a solution of the pharmaceutically active component compounds prepared in substantially the same manner as in Example 17, thereby yielding a cream preparation.

### Example 19:

A mixture of 200 grams of tannic acid and 40 grams of sodium hydrogen sulfite was ground in a mortar to pulverize into powders.

Separately, a mixture of 100 grams of potassium aluminum sulfate, 40 grams of lidocaine and 40 grams of borneol was ground in a mortar into powders and filtered to gather the powders having a predetermined range of particle sizes.

The two powders prepared in the manner as described hereinabove were combined together and mixed sufficiently to yield a uniform mixture. The resulting mixture was further divided finely to powders to which seed oil in turn was added to make the total weight 2,000 grams. The resulting mixture was mixed together and then molten to a sufficient extent. The molten material was then filled in containers for suppositories, thereby yielding a suppository preparation. The weight of one piece of the suppository was set to be 2 grams, and one piece of the suppository contains 0.2 gram of tannic acid, 0.1 gram of potassium aluminum sulfate, 0.04 gram of lidocaine and 0.04 gram of borneol.

### Example 20:

A mixture of 100 grams of tannic acid and 20 grams of sodium hydrogen sulfite was ground in a mortar to pulverize into powders.

Separately, a mixture of 50 grams of potassium aluminum sulfate, 20 grams of lidocaine and 20 grams of borneol was ground in a mortar into powders and filtered to gather the powders having a predetermined range of particle sizes.

The two powders prepared in the manner as described hereinabove were combined together and mixed sufficiently to yield a uniform mixture. The resulting mixture was further divided finely to powders to which molten cacao butter was added to make the entire weight 1,000 grams. The resulting mixture was mixed together and then molten to a sufficient extent. The molten material was then filled in containers for suppositories, thereby yielding a suppository preparation. The weight of one piece of the suppository was set to be 1.5 grams, and one piece of the suppository contains 0.15 gram of tannic acid, 0.075 gram of potassium aluminum sulfate, 0.03 gram of lidocaine and 0.03 gram of borneol.

### Example 21:

A mixture of 200 grams of tannic acid, 20 grams of sodium hydrogen sulfite and 100 ml of glycerin was heated and then ground in a mortar to pulverize into powders.

Separately, a mixture of 20 grams of potassium aluminum sulfate, 1 gram of penicillin, 40 grams of lidocaine and 20 grams of borneol was ground in a mortar into powders and filtered to gather the powders having a predetermined range of particle sizes.

The two powders prepared in the manner as described hereinabove were combined together and mixed sufficiently to yield a uniform mixture. The resulting mixture was further divided finely to powders to which witepsol was added to make the total weight 1,000 grams. The resulting mixture was mixed together and then molten to a sufficient extent. The molten material was then filled in capsules for suppository by 2 grams for each capsule.

### Example 22:

A mixture of 100 grams of tannic acid and 20 grams of sodium hydrogen sulfite was warmed and ground in a mortar to pulverize into powders.

Separately, a mixture of 20 grams of potassium aluminum sulfate, 40 grams of dibucaine hydrochloride, 20 grams of prednisolone and 20 grams of borneol was ground in a mortar into powders and filtered to gather the powders having a predetermined range of particle sizes.

The two powders prepared in the manner as described hereinabove were combined together and mixed sufficiently to yield a uniform mixture. The resulting mixture was further divided finely to powders to which vaseline was added to make the entire weight 1,000 grams and allowed to cool into a suppository form.

### Example 23:

A mixture of 100 grams of tannic acid, 20 grams of sodium hydrogen sulfite and 100 ml of glycerin was heated and ground in a mortar to pulverize into powders.

Separately, a mixture of 20 grams of potassium aluminum sulfate, 40 grams of polyoxyethylene sorbitan monooleate, sodium lauryl sulfate, 55 grams of triglyceride laurate, and 20 grams of lidocaine hydrochloride was ground in a mortar into powders and filtered to gather the powders having a predetermined range of particle sizes.

The two powders prepared in the manner as described hereinabove were combined together and mixed sufficiently yielding a uniform mixture while warming the mixture at 70°C and the uniform mixture was further mixed with a homogenizer to yield a uniform dispersion. The resulting mixture was filtered through a 200 mesh filter and then filled in gelatin soft suppository capsules by 2 grams for each capsule.

### Example 24:

A mixture of 100 grams of tannic acid and 20 grams of sodium hydrogen sulfite was placed in a mortar and ground to pulverize finely into uniform powders. Separately, a mixture of 30 grams of potassium aluminum sulfate, 20 gram of dibucaine hydrochloride and 20 grams of bismuth subnitrate was ground in a mortar into powders and filtered to gather the powders having a predetermined range of particle sizes. The two kinds of the powders prepared in the manner as described hereinabove were combined together and mixed sufficiently yielding a uniform mixture.

In addition, witepsol was dissolved at 40°C and sodium caprylate was added thereto, followed by mixing to a sufficient extent yielding a uniform dispersion. The resulting dispersion was then added to the uniform mixture separately prepared in the manner as described hereinabove, followed by filling the resulting mixture in suppository containers.

### Example 25:

A suppository preparation was prepared in substantially the same manner as in Example 24, using sodium caprate in place of sodium caprylate.

### Example 26:

A suppository preparation was prepared in substantially the same manner as in Example 24, using sodium laurate in place of sodium caprylate.

## Claims

1. A locally administrable preparation for treating affected tissues characterized in that said locally administrable preparation comprises a pharmaceutically effective component compound including tannic acid and potassium aluminum sulfate and a base component for said locally administrable preparation.

2. A locally administrable preparation for treating affected tissues as claimed in Claim 1; wherein said pharmaceutically effective component compound is contained at a rate of from 0.1% by weight to 30% by weight on the basis of the weight of said base component.

3. A locally administrable preparation for treating affected tissues as claimed in Claim 1 or 2; wherein said tannic acid is contained at a rate of from 10% to 90% by weight on the basis of said potassium aluminum sulfate.

4. A locally administrable preparation for treating affected tissues as claimed in any one of Claims 1 to 3, wherein said base component comprises a liquid base component, an organic solid, an oily substance or a surface active agent, each capable of solubilizing said pharmaceutically effective component compound.

5. A locally administrable preparation for treating affected tissues as claimed in any one of Claims 1 to 4, wherein said locally administrable preparation is in a form of ointment, cream, lotion, spray or suppository.
